# FASCICULE DE BREVET EUROPEEN

(11) **EP 2 331 549 B1**
(45) Date de publication et mention de la délivrance du brevet: **22.10.2014**
(21) Numéro de dépôt: 09748389.5
(22) Date de dépôt: 15.09.2009
(51) Int. Cl.: C07D 493/18, C07H 19/01

(54) **PROCEDE DE PREPARATION DU 1,6:2,3-DIANHYDRO-BETA-D-MANNOPYRANOSE**
VERFAHREN ZUR HERSTELLUNG VON 1,6:2,3-DIANHYDRO-BETA-D-MANNOPYRANOSE
METHOD FOR PREPARING 1,6:2,3-DIANHYDRO-BETA-D-MANNOPYRANOSE

(30) Priorité: 16.09.2008 FR 0805062
(43) Date de publication de la demande: 15.06.2011
(73) Titulaire: SANOFI, 75008 Paris (FR)
(72) Inventeur: GROSSI, Pierre, Jean, F-75013 Paris (FR); HOFF, Christian, F-75013 Paris (FR); ROVERA, Jean-Claude, F-75013 Paris (FR); SOLE, Raphaël, F-75013 Paris (FR)
(74) Mandataire: Blot, Philippe Robert Emile
(86) Numéro de dépôt international: PCT/FR2009/051729
(87) Numéro de publication internationale: WO 2010/031953

(56) Documents cités:
- BAILLIEZ V ET AL: "A practical large-scale access to 1,6-anhydro-[beta]-D-hexopyranoses by a solid-supported solvent-free microwave-assisted procedure" SYNTHESIS 2003 DE, no. 7, 2003, pages 1015-1017, XP002526685 ISSN: 0039-7881
- E. WOLFGANG HOLLA, VOLKER SINNWELL, WERNER KLAFFKE: "Two Syntheses of 3-Azido-3-deoxy-D-mannose" SYNLETT, 1992, pages 413-414, XP002526686
- MASUO AKAGI ET AL: "A new Synthesis of 1,6-Anhydro- D-glucopyranose (Levoglucosan)" CHEMICAL AND PHARMACEUTICAL BULLETIN, PHARMACEUTICAL SOCIETY OF JAPAN, TOKYO, no. 10, 1 janvier 1962 (1962-01-01), pages 905-909, XP009116335 ISSN: 0009-2363 cité dans la demande
- MILJKOVIC D ET AL: "A CONVENIENT ROUTE TO 6 FUNCTIONALIZED DERIVATIVES OF D GLUCAL" CARBOHYDRATE RESEARCH, vol. 193, 1989, pages 275-278, XP002526687 ISSN: 0008-6215

## Description

La présente invention se rapporte à un nouveau procédé de préparation du 1,6:2,3-dianhydro-β-D-mannopyranose, désigné ci-après « époxyde de Cerny » ou « composé (I) » et répondant à la formule suivante, dans laquelle les traits en gras représentent des liaisons situées au-dessus du cycle pyranosique : 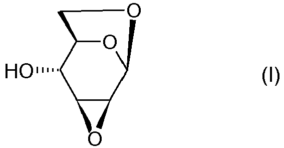 ou encore, selon une autre représentation :

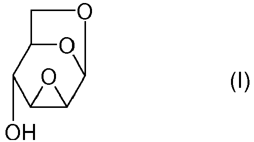

Le composé (I), et plus généralement les composés de la famille des 1,6:(2,3 et 3,4)-dianhydro-β-D-hexopyranoses, ont essentiellement été décrits par un chimiste tchèque, Miloslav Cerny. On trouve dans la littérature trois voies d'accès à l'époxyde de Cerny (I) à partir du composé 1 (1,6:3,4-dianhydro-4-O-tosyl-β-D-galactopyranose):

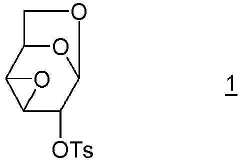

On obtient le composé 1 à partir du lévoglucosane 2 (ou 1,6-anhydro-β-D-glucopyranose), comme représenté ci-dessous (M. Cerny et al., Collect. Czech. Chem. Commun., 1961, vol. 26, p. 2542-2550) :

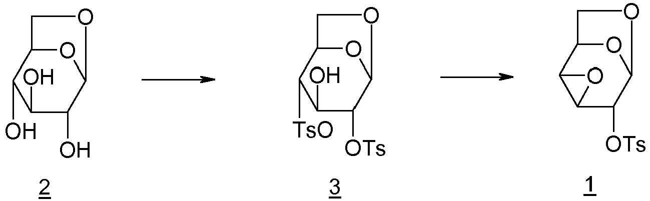

Le dérivé ditosylé 3 (1,6-anhydro-2,4-di-O-tosyl-β-D-glucopyranose) est obtenu sélectivement (80%). Les 20% restant sont essentiellement composés du dérivé tritosylé. Le rendement global pour la transformation du composé 2 en composé 1 est de 55%.

Les voies d'accès au lévoglucosane 2 sont nombreuses ; les plus utilisées industriellement sont, outre la pyrolyse de l'amidon et de la cellulose décrite depuis les années 1960, les cyclisations en milieu basique ou acide du D-glucose, représentées ci-dessous :

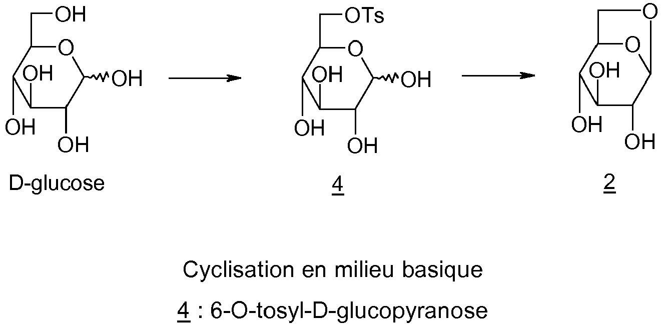

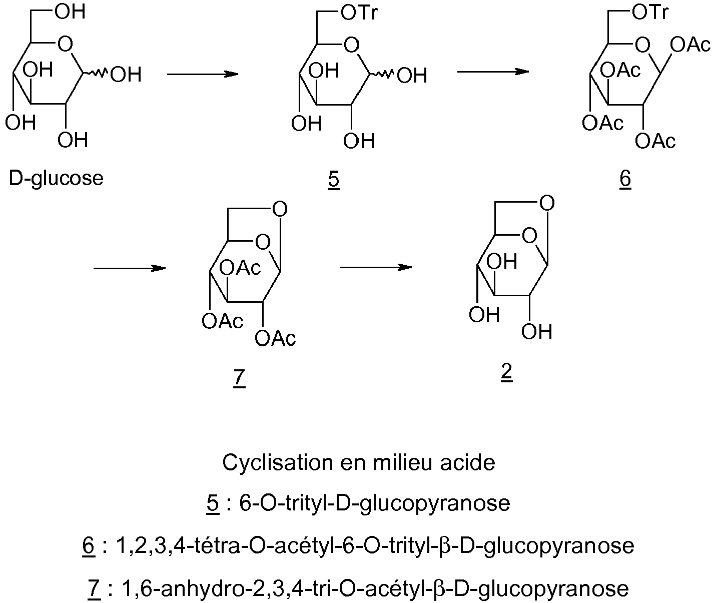

La cyclisation en milieu basique (M.A. Zottola et al., J. Org. Chem., 1989, vol. 54, p. 6123-6125 ; M. Akagi et al., Chem. Pharm. Bull., 1962, vol. 10, p. 905-909) se traduit par un rendement faible (15%). De plus il est nécessaire d'acétyler le lévoglucosane 2 brut pour permettre son isolement. Quant à la voie de cyclisation en milieu acide (M.V. Rao et al., Carbohydrate Research, 1987, vol. 162, 141-144 ; R.L. Wistler et al., Methods Carbohydr. Chem., 1972, vol. 6, p. 411-412 ; E. Zara-Kaczian *et al.,* 1982, vol. 111, no. 3, p. 271-283 ; E. Zara-Kaczian et al., Acta Chemica Acad. Scient. Hung., 1978, vol. 96, no. 3, p. 311-313), elle est décrite avec un meilleur rendement (70%), mais elle comporte deux étapes de plus.

Les trois voies d'accès à l'époxyde de Cerny (I) à partir du composé 1 sont les suivantes.

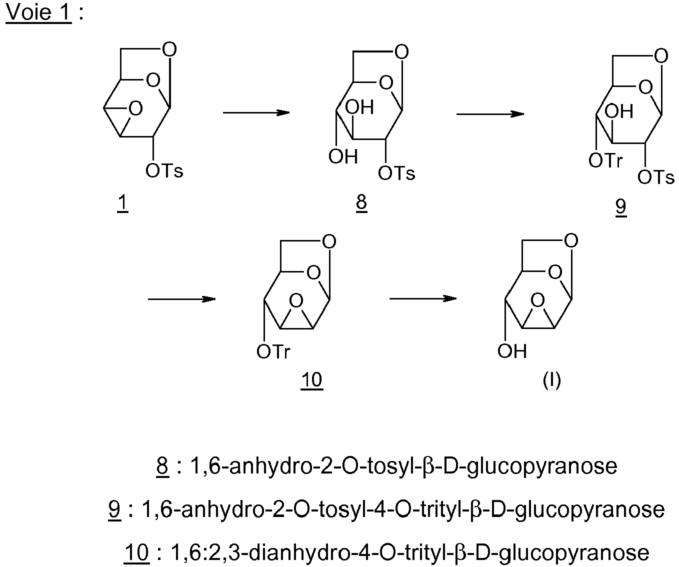

Outre le nombre d'étapes nécessaires pour parvenir à l'époxyde de Cerny (I), cet enchaînement comporte, entre autres, la difficulté d'hydrolyser sélectivement la fonction anhydro-3,4 lors de la première étape. L'hydroxyle en position 4 du dérivé monotosylé 8 est ensuite protégée par un groupement trityle (Tr) afin d'éviter la migration de l'époxyde lors de la cyclisation en présence d'éthylate de sodium (EtONa).

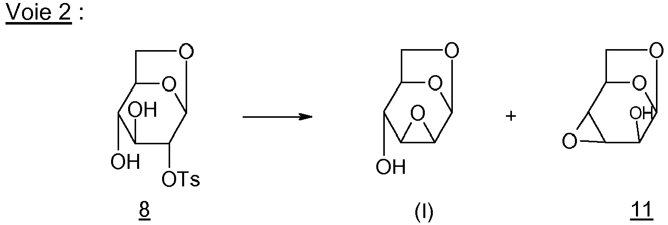

D'après M. Cerny et al. (Synthesis, 1972, 698-699), l'époxyde de Cerny (I) peut être obtenu à partir du dérivé 8 en présence de résine Amberlite IRA 400 / OH⁻. Toutefois, un contact prolongé avec la résine entraîne la migration de l'époxyde en position 3,4 et la formation du dérivé 11 (1,6:3,4-dianhydro-β-D-altropyranose). Il subsiste ainsi la difficulté d'obtenir sélectivement le composé (I). Le composé de départ 8 est lui-aussi difficile à obtenir sélectivement, comme mentionné précédemment.

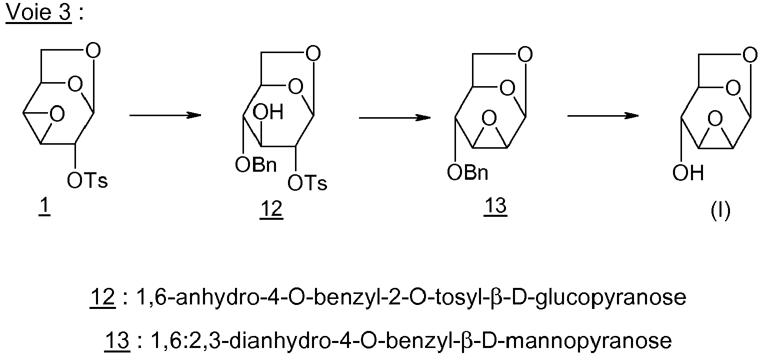

Cette variante permet la cyclisation en dérivé dianhydro 13 sans migration d'époxyde (T. Trnka et al., Collect. Czech. Chem. Commun., 1971, vol. 36, p. 2216-2225 ; M. Cerny et al., Collect. Czech. Chem. Commun., 1968, vol. 33, p. 1143-1156). Elle comporte néanmoins un grand nombre d'étapes pour fournir l'époxyde de Cerny (I) à partir du D-glucose.

En conclusion, les trois voies d'accès décrites ci-dessus pour la préparation de l'époxyde de Cerny (I) comptent respectivement 10, 8 et 9 étapes à partir du D-glucose (en utilisant, pour l'obtention du lévoglucosane 2, la cyclisation en milieu acide, qui est la voie décrite avec le meilleur rendement) et ont pour rendement global, respectivement pour les voies 1, 2 et 3, 0,5%, 10% et 13%.

Par ailleurs, V. Bailliez et al. ont décrit dans Synthesis, 2003, No. 7, 1015-1017 une voie d'accès au 1,6:3,4-dianhydro-β-D-altropyranose, qui s'accompagne d'une formation minoritaire de 1,6:2,3-dianhydro-β-D-mannopyranose en tant que sous-produit. Selon ces auteurs, l'époxyde de Cerny peut être formé à partir d'un précurseur préalablement cyclisé entre les positions 1 et 6, ou bien un précurseur N-1 de l'époxyde de Cerny acétylé en position 4 peut être obtenu, à hauteur de 5%, en plusieurs étapes à partir de 1,3,4-tri-O-acétyl-2,6-di-O-tosyl glucose soumis à de l'alumine, irradiation sous midroondes et per-O-acétylation.

E.W. Holla *et al.* ont également décrit, dans Synlett, 1992, 413-414, l'utilisation d'un précurseur préalablement cyclisé entre les positions 1 et 6 pour former ensuite l'époxyde de Cerny par réaction avec le méthanolate de sodium, ledit époxyde étant obtenu en mélange avec le 1,6:3,4-dianhydro-β-D-altropyranose.

Compte tenu des coûts de main d'oeuvre et des matières premières, et pour une obtention du composé (I) à l'échelle industrielle, il est nécessaire d'envisager une synthèse plus courte, offrant un meilleur rendement, et donc plus rentable. Les Inventeurs ont maintenant trouvé une voie d'accès au composé (I) en trois étapes à partir du D-glucose, qui satisfait aux besoins sus-mentionnés.

Le procédé selon l'invention comprend les étapes représentées ci-dessous dans le schéma 1.

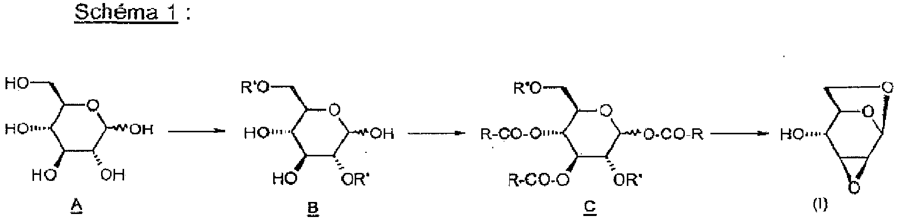

L'invention a ainsi pour objet un procédé de préparation du composé (I), caractérisé en ce qu'il comprend une étape de cyclisation du composé C dans un mélange alcool/alcoolate, en conditions anhydres.

Dans le composé C, R représente un groupe alkyle comprenant de 1 à 4 atomes de carbone, par exemple un groupe méthyle, et R' représente un groupe activant, par exemple un groupe tosyle, mésyle ou benzènesulfonyle.

Au sens de la présente invention, et sauf mention différente dans le texte, on entend par :
- « alkyle » : un groupe aliphatique saturé, linéaire ou ramifié, par exemple un groupe méthyle ;
- « alcool » : un composé de formule alkyl-OH, dans lequel le groupe alkyle est tel que défini ci-dessus et comprend de 1 à 3 atomes de carbone, par exemple le méthanol ;
- « alcoolate » : la base conjuguée de l'alcool tel que défini ci-dessus, c'est-à-dire l'anion répondant à la formule alkyl-O⁻, portant un contre-ion de métal alcalin tel que le sodium ;
- « mélange alcool/alcoolate » : un mélange d'un alcool avec l'alcoolate correspondant, par exemple un mélange méthanol/méthanolate de sodium (CH₃OH/CH₃ONa) ;
- « agent activant » : un agent permettant le départ du groupe partant -OR' et favorisant la réaction de cyclisation entre les positions 1 et 6 du composé C, par exemple un halogénure de tosyle, de mésyle, de benzènesulfonyle ou de dérivé de benzènesulfonyle, tel qu'un halogénure de p-halogéno-benzylesulfonyle.

Selon l'invention, la cyclisation du composé C est avantageusement réalisée à l'aide de 2 à 3 équivalents d'alcoolate (exprimés par rapport à la quantité molaire de composé C), de préférence 2,2 équivalents.

L'invention a également pour objet un procédé de préparation du composé (I), caractérisé en ce qu'il comprend une étape d'acylation du composé B (dans lequel R' est tel que défini ci-dessus), permettant d'obtenir le composé C, suivie d'une étape de cyclisation du composé C dans un mélange alcool/alcoolate, telle que définie précédemment.

L'étape d'acylation du composé B est réalisée à l'aide d'un agent acylant permettant l'introduction des groupements R-CO- dans le composé C ; un tel agent acylant peut par exemple consister en un anhydride d'acide, tel que l'anhydride acétique, ou en un chlorure d'acyle. On utilise avantageusement au moins trois équivalents d'agent acylant par rapport au composé B.

Selon un mode de réalisation de l'invention, le composé C est tel que R représente un groupe méthyle. Dans ce cas, la réaction d'acylation du composé B consiste en une réaction d'acétylation, réalisée par exemple à l'aide d'anhydride acétique, dans un solvant tel que le dichlorométhane.

L'invention a également pour objet un procédé de préparation du composé (I), caractérisé en ce qu'il comprend une étape d'activation du composé A (D-glucose), permettant d'obtenir le composé B, puis une étape d'acylation du composé B, suivie d'une étape de cyclisation du composé C, obtenu à l'issue de l'étape précédente, dans un mélange alcool/alcoolate en conditions anhydres.

L'étape d'activation du composé A peut être réalisée à l'aide d'un agent activant tel que défini précédemment. On utilise ainsi avantageusement du chlorure de tosyle, dans un solvant tel que la pyridine.

Le procédé selon l'invention permet l'obtention sélective du composé (I) (1,6:2,3-dianhydro-β-D-mannopyranose) en trois étapes à partir du D-glucose, notamment de par la faible basicité du milieu lors de la réaction de cyclisation de l'intermédiaire C, des conditions réactionnelles anhydres et du nombre d'équivalents de méthanolate de sodium utilisé.

Le composé (I) est obtenu, selon le procédé de l'invention, avec une sélectivité d'au moins 90% à partir de l'intermédiaire C. Le rendement chimique calculé sur produit isolé, c'est-à-dire après les diverses étapes de lavage, filtration, élimination de solvant requises pour son isolement, telles qu'il est classique de les mettre en oeuvre en chimie organique, est d'au moins 60%.

Les réactions de transformation du D-glucose en intermédiaire B, puis de formation de l'intermédiaire C à partir du composé B, présentent un rendement chimique d'au moins 50% et 80%, respectivement.

L'invention est illustrée à l'aide des exemples qui suivent, qui détaillent un procédé de préparation du composé (I) conformément à l'invention, suivant le schéma 2 ci-dessous. Dans ces exemples, on utilise les abréviations suivantes : Me : méthyle ; Et : éthyle ; Ac : acétyle ; CCM : Chromatographie sur Couche Mince ; HPLC : High Performance Liquid Chromatography ; DMAP : 4-diméthylaminopyridine.

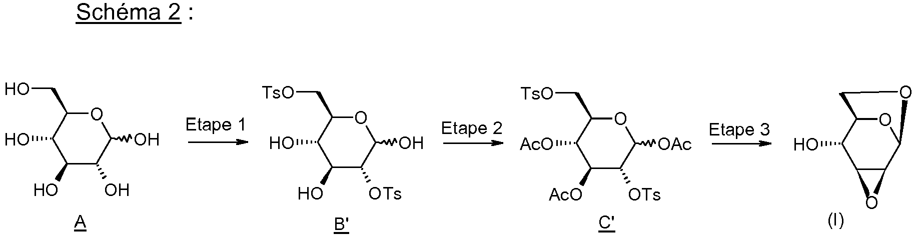

### Etape 1 : Préparation du composé B' (2,6-di-O-tosyl-glucopyranose)

Dans un réacteur de 6 litres sous azote, on introduit successivement 250 g (1,38 mol) de D-glucose (composé A) et 1240 ml de pyridine anhydre. On refroidit à -10°C, sous agitation. En parallèle, dans un deuxième réacteur, on prépare une solution pyridinique de chlorure de tosyle en dissolvant 529 g (2,78 mol) de chlorure de tosyle dans 1760 ml de pyridine anhydre. On coule ensuite la solution de chlorure de tosyle sur la solution de glucose préparée précédemment. On maintient sous agitation pendant 17 h à -10°C. On contrôle le taux d'avancement de la réaction par CCM (éluant CH₂Cl₂/MeOH 9/1 V/V pour la quantification des dérivés di- et tritosylés ; éluant CHCl₃/EtOH/AcOH/H₂O 48/40/8/4 V/V pour le suivi du D-glucose) et par HPLC, dans des conditions usuelles.

On concentre par distillation à 45-50°C sous vide. Lorsque le milieu s'épaissit et que le volume résiduel est d'environ 1000 ml, on charge 750 ml d'eau déminéralisée, on homogénéise et on distille ensuite environ 750 ml du mélange à une température d'environ 45-50°C sous environ 20 mm Hg (opération d'échange de solvant par distillation). On répète l'opération de distillation jusqu'à élimination de la pyridine. Lorsque la température du réacteur est abaissée à 20°C, on ajoute 1000 ml de dichlorométhane et on homogénéise sous agitation. On introduit successivement 1000 ml d'eau déminéralisée et 100 ml d'acide chlorhydrique, on agite encore 30 minutes et après décantation on élimine la phase aqueuse acide. Ces manipulations sont répétées jusqu'à obtention d'un pH de la phase aqueuse voisin de 1. On introduit au mélange précédent une solution composée de 1000 ml d'eau déminéralisée et de 100 g de NaCl. Le mélange est agité encore 30 minutes, on laisse décanter et on élimine ensuite la phase aqueuse. Ces manipulations sont répétées jusqu'à obtention d'un pH de la phase aqueuse de 5,0 à 5,5. On élimine ensuite le dichlorométhane par distillation à une température de 45°C jusqu'à obtention d'un volume résiduel de 625 ml, puis on élimine l'eau par quatre opérations de distillation à cette même température, avec à chaque fois 625 ml de dichlorométhane.

Le rendement de l'étape 1 est de 64%.

### Etape 2 : Préparation du composé C' (ou 1,3,4-tri-O-acétyl-2,6-di-O-tosyl glucose)

On reprend le concentrat obtenu à l'issue de l'étape précédente par 625 ml de dichlorométhane (ajustement du volume réactionnel à 1300 ml). On ajoute 24 g de DMAP, puis 616 g d'anhydride acétique en 1 h 30 min., à une température de 20°C. On chauffe le milieu réactionnel à 43°C et on maintient sous agitation pendant environ 3 h. Le taux d'avancement de la réaction est contrôlé par CCM (éluant : toluène/AcOEt 90/30 V/V). On refroidit le milieu réactionnel à 20°C et on introduit 1000 ml d'eau déminéralisée. On agite le milieu réactionnel pendant 30 min., on laisse décanter et la phase aqueuse acide est éliminée. On ajoute ensuite 1000 ml d'eau déminéralisée et 100 g de Na₂CO₃. A nouveau, on agite le milieu réactionnel pendant 30 min., on laisse décanter et la phase aqueuse acide est éliminée. On procède une nouvelle fois à l'ajout de 1000 ml d'eau déminéralisée, suivi d'agitation pendant 30 min., décantation et élimination de la phase aqueuse. On concentre ensuite la phase organique à un volume compris entre 875 ml et 1000 ml par élimination du dichlorométhane et distillation sous vide, puis on élimine le dichlorométhane par distillation sous vide au méthanol, en utilisant à chaque fois 500 ml de méthanol. On ajuste le volume réactionnel à 2000 ml par ajout de méthanol, on refroidit le milieu réactionnel sous agitation à 0°C et on maintient cette température pendant 3 h. On filtre le précipité obtenu. On lave ensuite 3 fois le gâteau obtenu par clairçage avec 250 ml de méthanol à 0°C. Le produit ainsi obtenu (composé C') est séché sous pression réduite à 50°C et à poids constant.

Le rendement de l'étape 2 est de 95%, le composé C' obtenu présentant une pureté de 93,4%, mesurée par titre HPLC.

### Etape 3 : Préparation du composé (I)

Dans un réacteur de 6 litres, on introduit successivement 604 g du composé C' (0,98 mol) et 3600 ml de méthanol. On agite à une température de 20°C. On ajoute ensuite 375,8 g de méthanolate de sodium (MeONa) 30%/méthanol, soit 2,12 mol de méthanolate. Le méthanol utilisé dans cette étape réactionnelle est anhydre (il contient moins de 200 ppm d'eau). On maintient sous agitation pendant 5 h à 20°C. On refroidit ensuite le milieu réactionnel à 0°C, puis on ajuste le pH à 6,5 par ajout de 13,5 ml d'acide chlorhydrique (36%).

La réaction de cyclisation du composé C' est sélective et se traduit par une conversion d'au moins 90% du composé C' en composé (I). Des étapes de lavage et d'élimination de solvants sont ensuite nécessaires pour obtenir le composé (I) sous forme isolée.

On concentre sous pression réduite à une température de 30°C jusqu'à obtenir un volume résiduel de 980 ml, puis on ajoute 1600 ml d'acétate d'éthyle. On concentre ensuite sous pression réduite à une température de 60°C jusqu'à obtenir un volume résiduel de 966 ml. On introduit 980 ml d'acétate d'éthyle et on concentre sous pression réduite à une température de 30°C jusqu'à obtenir un volume résiduel de 980 ml. On introduit à nouveau 300 ml d'acétate d'éthyle, puis on concentre sous pression réduite à une température de 30°C jusqu'à obtenir un volume résiduel de 980 ml (opérations à répéter encore deux autres fois). L'élimination du méthanol est suivie par l'indice de réfraction des dernières gouttes de distillat.

Lorsque tout le méthanol est remplacé par de l'acétate d'éthyle, on refroidit en 30 min. le milieu réactionnel à 0°C et on maintient sous agitation pendant 1 h à cette température. On filtre la suspension obtenue, puis on lave par clairçage le gâteau, 4 fois et avec à chaque fois 300 ml d'acétate d'éthyle à 0°C. Les jus de filtration ainsi obtenus sont réunis. On concentre sous pression réduite à une température de 25-30°C jusqu'à obtenir un volume résiduel de 780 ml.

Le rendement chimique de l'étape 3 est de 75 %.

Les spectres RMN du proton et du carbone 13 du composé (I) sont enregistrés sur un appareil Bruker 300 MHz. Les déplacements chimiques sont exprimés par rapport au tétraméthylsilane, à 0,01 ppm près pour le spectre du proton et à 0,1 ppm près pour le spectre du carbone 13. Les constantes de couplage sont données en valeur absolue en Hz à 0,5 Hz près.

RMN ¹H (CDCl₃) : 2,67 (d, 1 H, OH, J _{4,OH} 5,5 Hz), 3,12 (d, 1 H, H₃, J _{2,3} 3,4 Hz), 3,42 (dd, 1 H, H₂, J _{2,3} = J _{2,1} = 3,0 Hz), 3,69 à 3,77 (m, 2H, H₆, H_{6'}), 3,89 (d, 1 H, H₄, J _{4,OH} 5,5 Hz), 4,40 (dm, 1 H, H₁, J _{1,2} 3,0 Hz).

RMN ¹³C : 49,3: C₃; 54,3: C₂; 65,6: C_{6,6'}; 67,1: C₄; 97,7: C₁; 74,2: C₅.

## Revendications

1. Procédé de préparation du composé (I) : 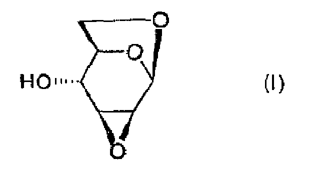 **caractérisé en ce qu'**il comprend une étape de cyclisation du composé C: 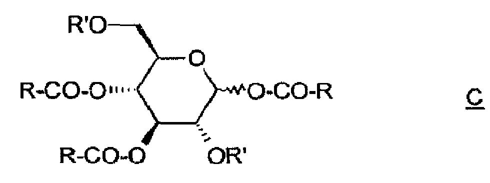 dans lequel R représente un groupe alkyle comprenant de 1 à 4 atomes de carbone et R' représente un groupe activant, dans un mélange alcool/alcoolate en conditions anhydres.

2. Procédé selon la revendication 1, **caractérisé en ce que** le composé C est tel que R représente un groupe méthyle.

3. Procédé selon la revendication 1 ou la revendication 2, **caractérisé en ce que** le composé C est tel que R' représente un groupe tosyle, mésyle ou benzènesulfonyle.

4. Procédé selon l'une quelconque des revendications 1 à 3, **caractérisé en ce que** le composé C répond à la formule C' : 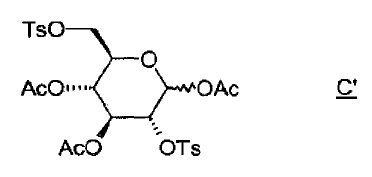 dans laquelle Ac représente un groupe acétyle et Ts représente un groupe tosyle.

5. Procédé selon l'une quelconque des revendications 1 à 4, **caractérisé en ce que** l'étape de cyclisation est effectuée dans un mélange méthanol/méthanolate de sodium.

6. Procédé selon l'une quelconque des revendications 1 à 5, **caractérisé en ce que** l'étape de cyclisation est réalisée à l'aide de 2 à 3 équivalents d'alcoolate.

7. Procédé selon l'une quelconque des revendications 1 à 6, **caractérisé en ce qu'**il comprend :
- une étape d'acylation du composé B : 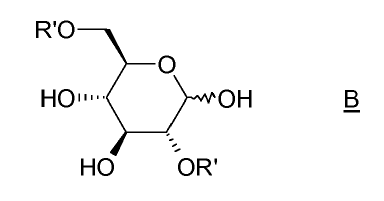 dans lequel R' est tel que défini dans la revendication 1, permettant d'obtenir le composé C tel que défini dans la revendication 1,
- suivie d'une étape de cyclisation du composé C, telle que définie dans l'une quelconque des revendications 1 à 6.

8. Procédé selon la revendication 7, **caractérisé en ce que** le composé B est tel que R' représente un groupe tosyle et répond à la formule B' :

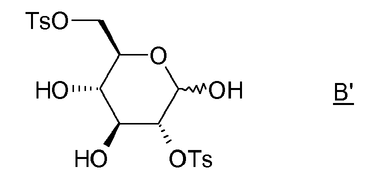

9. Procédé selon la revendication 7 ou la revendication 8, **caractérisé en ce que** l'étape d'acylation est une réaction d'acétylation.

10. Procédé selon la revendication 9, **caractérisé en ce que** la réaction d'acétylation est effectuée à l'aide d'anhydride acétique.

11. Procédé selon l'une quelconque des revendications 1 à 10, **caractérisé en ce qu'**il comprend :
- une étape d'activation du composé A : 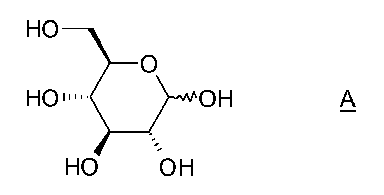 permettant d'obtenir le composé B tel que défini dans la revendication 7,
- suivie d'une étape d'acylation du composé B, telle que définie dans la revendication 7 ou la revendication 9, et permettant d'obtenir le composé C tel que défini dans la revendication 1,
- suivie d'une étape de cyclisation du composé C, telle que définie dans l'une quelconque des revendications 1 à 6.

12. Procédé selon la revendication 11, **caractérisé en ce que** l'étape d'activation du composé A est réalisée à l'aide d'un halogénure de tosyle, de mésyle ou de benzènesulfonyle.

## Patentansprüche

1. Verfahren zur Herstellung von Verbindung (I): 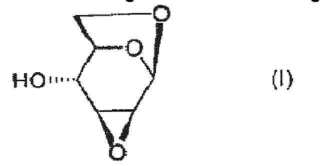 **dadurch gekennzeichnet, dass** es einen Schritt der Cyclisierung von Verbindung C: 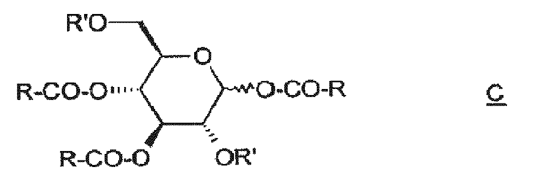 worin R für eine Alkylgruppe mit 1 bis 4 Kohlenstoffatomen steht und R' für eine Aktivierungsgruppe steht, unter wasserfreien Bedingungen in einer Alkohol/Alkoholat-Mischung umfasst.

2. Verfahren nach Anspruch 1, **dadurch gekennzeichnet, dass** die Verbindung C so beschaffen ist, dass R für eine Methylgruppe steht.

3. Verfahren nach Anspruch 1 oder Anspruch 2, **dadurch gekennzeichnet, dass** die Verbindung C so beschaffen ist, dass R' für eine Tosyl-, Mesyl- oder Benzolsulfonylgruppe steht.

4. Verfahren nach einem der Ansprüche 1 bis 3, **dadurch gekennzeichnet, dass** die Verbindung C der Formel C' entspricht: 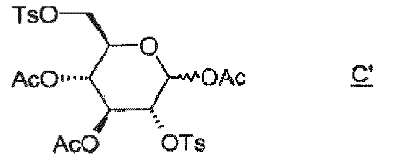 worin Ac für eine Acetylgruppe steht und Ts für eine Tosylgruppe steht.

5. Verfahren nach einem der Ansprüche 1 bis 4, **dadurch gekennzeichnet, dass** man den Cyclisierungsschritt in einer Methanol/Natriummethanolat-Mischung durchführt.

6. Verfahren nach einem der Ansprüche 1 bis 5, **dadurch gekennzeichnet, dass** man den Cyclisierungsschritt mit Hilfe von 2 bis 3 Äquivalenten Alkoholat durchführt.

7. Verfahren nach einem der Ansprüche 1 bis 6, **dadurch gekennzeichnet**, es Folgendes umfasst:
- einen Schritt der Acylierung von Verbindung B: 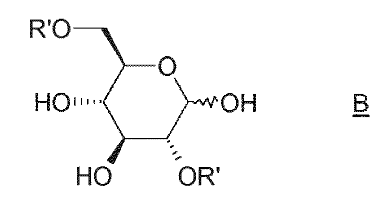 worin R' wie in Anspruch 1 definiert ist, zu Verbindung C, die wie in Anspruch 1 definiert ist,
- gefolgt von einem Schritt der Cyclisierung von Verbindung C, der wie in einem der Ansprüche 1 bis 6 definiert ist.

8. Verfahren nach Anspruch 7, **dadurch gekennzeichnet, dass** die Verbindung B so beschaffen ist, dass R' für eine Tosylgruppe steht, und der Formel B' entspricht:

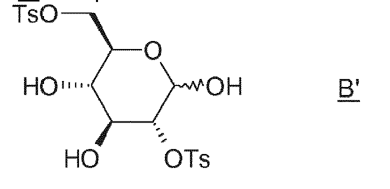

9. Verfahren nach Anspruch 7 oder Anspruch 8, **dadurch gekennzeichnet, dass** es sich bei dem Acylierungsschritt um eine Acetylierungsreaktion handelt.

10. Verfahren nach Anspruch 9, **dadurch gekennzeichnet, dass** man die Acetylierungsreaktion mit Hilfe von Essigsäureanhydrid durchführt.

11. Verfahren nach einem der Ansprüche 1 bis 10, **dadurch gekennzeichnet, dass** es Folgendes umfasst:
- einen Schritt der Aktivierung von Verbindung A: 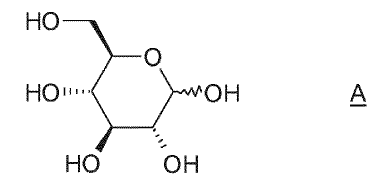 zu Verbindung B, die wie in Anspruch 7 definiert ist,
- gefolgt von einem Schritt der Acylierung von Verbindung B, der wie in Anspruch 7 oder Anspruch 9 definiert ist, zu Verbindung C, die wie in Anspruch 1 definiert ist,
- gefolgt von einem Schritt der Cyclisierung von Verbindung C, der wie in einem der Ansprüche 1 bis 6 definiert ist.

12. Verfahren nach Anspruch 11, **dadurch gekennzeichnet, dass** man den Schritt der Aktivierung von Verbindung A mit Hilfe eines Tosyl-, Mesyl- oder Benzolsulfonylhalogenids durchführt.

## Claims

1. Process for the preparation of compound (I): 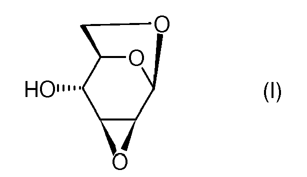 **characterized in that** it comprises a stage of cyclisation of compound C: 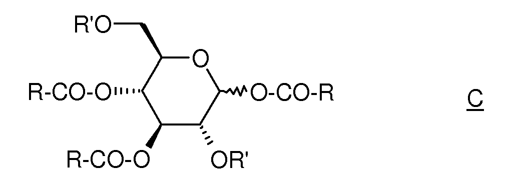 in which R represents an alkyl group comprising from 1 to 4 carbon atoms, and R' represents an activating *group,* in a alcool/alcoholate mixture under anhydrous conditions.

2. Process according to claim 1, **characterized in that** compound C is such that R represents a methyl group.

3. Process according to claim 1 or claim 2, **characterized in that** compound C is such that R' represents a group selected from tosyl, mesyl and benzenosulphonyl group,

4. Process according to anyone of claims 1 to 3, **characterized in that** compound C corresponds to the formula C': 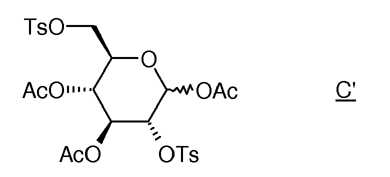 in which Ac represents an acetyl group and Ts represents a tosyl group.

5. Process according to anyone of claims 1 to 4, **characterized in that** the cyclisation stage is carried out a methanol/methanolate mixture.

6. Process according to anyone of claims 1 to 5, **characterized in that** the cyclisation stage is carried out using from two to three equivalents of methanolate.

7. Process according to any one of claims 1 to 6, **characterized in that** it comprises:
a stage of acylation of compound B: 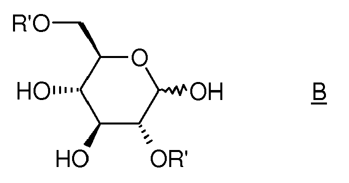 in which R' is as defined in claim 1, making it possible to obtain compound C as defined in claim 1,
-followed by a stage of cyclisation, as defined in any one of claims 1 to 6, of compound C.

8. Process according to claim 7, **characterized in that** compound B is such that R' represents a tosyl group and corresponds to the formula B':

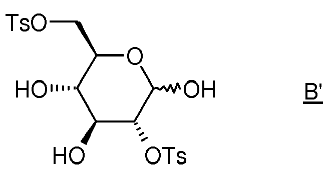

9. Process according to claim 7 or claim 8, **characterized in that** the acylation stage is an acetylation reaction.

10. Process according to claim 9, **characterized in that** the acetylation reaction is carried out using acetic anhydride.

11. Process according to any one of claims 1 to 10, **characterized in that** it comprises:
a stage of activation of compound A: 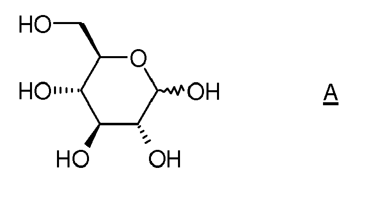 making it possible to obtain compound B as defined in claim 7,
- followed by a stage of acylation, as defined in claim 7 or claim 9, of compound B, making it possible to obtain compound C as defined in claim 1,
- followed by a stage of cyclisation, as defined in any one of claims 1 to 6, of compound C.

12. Process according to claim 11, **characterized in that** the stage of activation of compound A is carried out using a tosyl, mesyl or benzenesulphonyl halide.
